# EUROPEAN PATENT SPECIFICATION

(11) **EP 2 326 247 B1**
(45) Date of publication and mention of the grant of the patent: **13.03.2013**
(21) Application number: 08793567.2
(22) Date of filing: 29.08.2008
(51) Int. Cl.: A61B 5/145, A61B 5/151, A61B 5/00

(54) **ONE TOUCH LASER LANCING TYPE BLOOD GLUCOSE MEASUREMENT DEVICE, STRIP AND THE METHOD USING IT**
MIT EINEM TASTENDRUCK BEDIENBARES BLUTZUCKERMESSGERÄT VOM LASERLANZETTEN-TYP, STREIFEN UND ANWENDUNGSVERFAHREN DAMIT
DISPOSITIF DE MESURE DU GLUCOSE SANGUIN DE TYPE AUTOPIQUEUR LASER À UNE TOUCHE, BANDE ET SON PROCÉDÉ D UTILISATION

(30) Priority: 27.08.2008 KR 20080083705
(43) Date of publication of application: 01.06.2011
(73) Proprietor: Isotech Co., Ltd, Daejeon-si 305-510 (KR)
(72) Inventor: CHOI, Kee Jung, Daejeon-si 305-769 (KR)
(74) Representative: Cabinet Plasseraud
(86) International application number: PCT/KR2008/005067
(87) International publication number: WO 2010/024484

(56) References cited:
- WO-A1-98/04201
- WO-A1-2007/108513
- WO-A1-2007/119900
- WO-A2-2005/107594
- KR-B1- 100 781 323
- US-A- 5 947 957
- US-B1- 6 233 269

## Description

### [Technical Field]

The present invention relates to a one touch laser lancing type blood glucose measurement device, a strip, and the method using it. In particular, the present invention relates to a one touch laser lancing type blood glucose measurement device, a strip, and the method using it, which performs the blood gathering and the blood glucose measurement at the same time, that by using laser beams generated in a laser module, the laser beams are irradiated to a finger passing a hole of a strip with a focusing lens converging the laser beams, a protect window, and the hole to gather blood, then the blood is absorbed into the strip for measuring the blood glucose.

### [Background Art]

Recently, International Diabetes Federation (IDF) has warned that diabetics would be over about 360 millions around the globe, and the diabetics increase about 8.6% for every year, and thereby solutions by the government are required. For diagnosing diabetes, the density of glucoses in blood plasma of blood components is measured, and the management of the blood sugar is very important.

General blood-gathering and blood glucose measurement device pricks a skin surface by using a lancing device with a lancet having a metal needle shape. Thus, diabetes feel a pain and have a scar and large blood amount that is surplus is gathered and is dropped to a strip end. Then, a strip is inserted into a blood glucose measurement device separately formed, to measure blood sugar level.

As described above, for measuring the blood sugar level, the lancet, the lancing device, the blood glucose measurement device, the strip, and alcohol cottons need.

However, by the excessive TV watching and an irregular diet habit, the children suffering from the diabetes increase. The lancet having the needle causes pain and fear from the children and the adult.

In addition, a diabetic relying on insulin injections who measures the blood sugar level 2 to 4 times every one day must have a blood glucose measure device, but it is onerous to carry the various components of the blood glucose measurement device.

For solving the problems, an apparatus having the blood glucose measurement device using the lancet and the lancing device and the blood-gathering device using the laser beams has been developed. The blood is gathered using the laser beams in one end of the apparatus and is dropped into the strip, and then the strip is inserted into a strip connector to measure the blood sugar level in another end of the apparatus.

However, even though the apparatus having both the blood-gathering function and the blood sugar level measuring function, the blood should be gathered by inserting a disposal cap into one end of the apparatus, the strip is inserted into another end of the apparatus for measuring the blood sugar level. Thus, the usage of the apparatus is not easy. For avoiding the pollution of the blood glucose measurement device, the disposal cap covered with a film is used, but have to be carried.

For instance, document WO-2007/119900 discloses a solution for lancing the finger of a user wherein the finger is irradiated by a laser beam from a laser module. A strip on which the user can presses his irradiated finger is provided to deposit a drop of blood. However, the strip is apart from the laser module so that the finger has to be at different positions for skin lancing and for blood-gathering. Thus, a gesture is needed from the user to retire his finger from the laser module and then to press the strip.

Since the apparatus installed the blood glucose measurement device and the blood-gathering device on both ends, respectively, the volume of the apparatus increases. Thereby, when the diabetic relying on insulin injections is a child, the controlling of the apparatus is difficult due to the large volume.

### [DETAILED DESCRIPTION]

### [Technical Problem]

The object of the present invention provides a one touch laser lancing type blood glucose measurement device, a strip, and the method using it performing a blood-gathering process, a process sucking the blood with the strip, and a process measuring the blood glucose level by a one button operation at the same time in a blood glucose measurement device and the strip.

The another object of the present invention provides a one touch laser lancing type blood glucose measurement device, a strip, and the method using it decreasing volume by having a blood-gathering device and a blood glucose measurement device.

The further another object of the present invention provides a strip forming a film on an upper end of a strip hole for avoiding the pollution.

### [Technical Solution]

A one touch laser lancing type blood glucose measurement device according to the present invention includes a laser module generating laser beams and irradiating the laser beams in one direction; a focusing lens converging the laser beams generated from the laser module and disposed in a laser irradiation direction; a protect window disposed in a laser direction progressing through the focusing lens and protecting the focusing lens from foreign bodies; a controlling unit disposed on a position close to a lateral side of the laser module and controlling a blood measure and the laser beams; a strip connector connected to the controlling unit and formed in a position on which a strip hole and the protect window are coincided with each other, and inserting a strip; a body unit containing the laser module, the focusing lens, the protect window, the controlling unit, and the strip connector; an interlock disposed on the outside of the body unit, positioned on the upper or lower side of the strip inserted into the strip connector, forming a hole, and changing the laser module by pressing with a finger into a ready state; a button unit formed on a lateral side different from or an opposite side to the interlock holder and operating the laser module, and, into the ready state, the interlock hole, the strip hole, the protect window and the focusing lens are disposed on a straight line.

The present invention further includes a finger interlock formed on the outside of the body unit, preventing the laser beams irradiated from the laser module from exposing to an external, and having a shape, which a finger is inserted into and a first sensor making a user sense the finger interlock' s existence.

The present invention further includes a second sensing unit pressing the interlock holder to sense the ready state of the laser module.

The present invention further includes an adapted strip comprising a reagent unit; an electrode unit inserted into a strip connector to transfer blood information to the blood glucose measurement device; and a strip hole passing laser beams irradiated from the laser module to irradiate the laser beams.

The present invention further includes an adapted strip comprising a poly propylene film included on the upper of the strip hole to prevent the blood glucose measurement device from polluting.

The reagent unit measures any one of glucose which is a ferment, anti-insulin, and HbAIc.

A method for measuring blood glucose using a one touch laser lancing type blood glucose measurement device according to the present invention includes a first process inserting a strip into a strip connector; a second process inserting a finger into an upper of an interlock holder and into the inner of the finger lock holder; a third process changing into a ready state by pressing the interlock holder; a third process changing into a ready state by pressing the interlock holder; a fifth process converging the generated laser beams into a focusing lens, passing a protect window, a strip hole, and an interlock holder, and then irradiating the laser beams into the inserted finger; a sixth process making blood oozed by the laser beams irradiated into the finger be absorbed in the strip; a seventh process transmitting data for the absorbed blood to a controlling unit; and an eighth process outputting blood analyzed data analyzed by the controlling unit to a displaying unit.

### [Advantageous Effects]

A one touch laser lancing type blood glucose measurement device, a strip, and the method using it according to the present invention performs a blood-gathering process, a process sucking the blood with the strip, and a process measuring the blood glucose level by a one button operation at the same time in a blood glucose measurement device and the strip.

The present invention is a mixed construction having a blood-gathering device and a blood glucose measurement device installed on one side, and thereby the volume decreases. Thus, the carrying of the one touch laser lancing type blood glucose measurement device is easy.

The present invention blocks dusts etc. absorbed on a focusing lens by irradiating high temperature laser beams generated in blood-gathering by laser beams and exactly measuring the blood sugar level using a small blood.

### [Brief Description of the Drawings]

FIG. 1 is a diagram representing a one touch laser lancing type blood glucose measurement device according to the present invention.
FIG. 2 shows a diagram representing the blood-gathering using a one touch laser lancing type blood glucose measurement device according to the present invention.
FIG. 3 shows that blood gathered using a one touch laser lancing type blood glucose measurement device according to the present invention is absorbed into a strip.
FIG. 4 is a diagram showing a strip used in a one touch laser lancing type blood glucose measurement device according to the present invention.
FIG. 5 is a flow chart of a blood glucose measurement method using a one touch laser lancing type blood glucose measurement according to the present invention.

### <Description of the reference numerals in the drawings>

- 101 :: laser module
- 103 :: protect window
- 105 :: interlock holder
- 107 :: finger interlock
- 109 :: body unit
- 111 :: displaying unit
- 113 :: blood
- 401 :: electrode unit
- 403 :: poly propylene film

### [Best Mode]

Hereinafter, embodiments of the present invention will be described in detail referring to drawings.

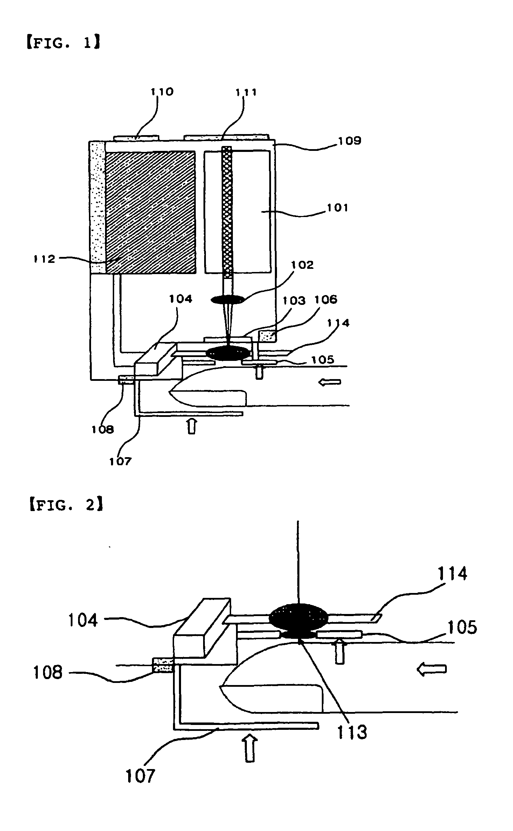

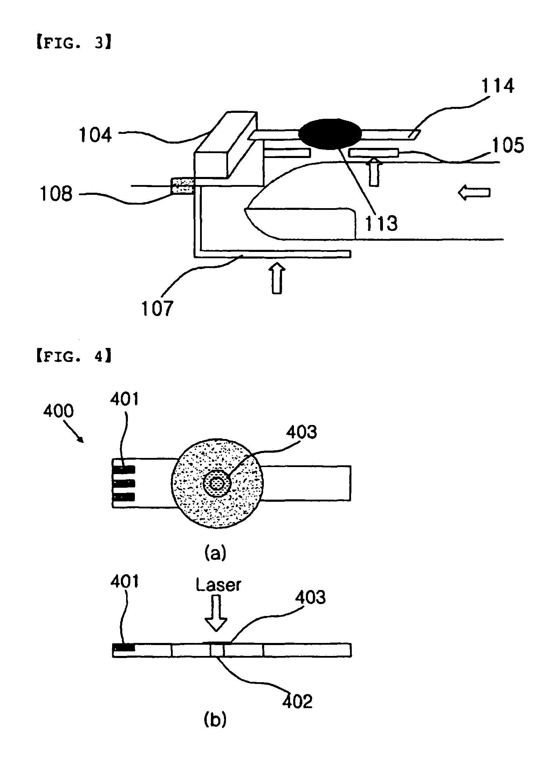

FIG. 1 is a diagram representing a one touch laser lancing type blood glucose measurement device according to the present invention, FIG. 2 shows a diagram representing the blood-gathering using a one touch laser lancing type blood glucose measurement device according to the present invention, and FIG. 3 shows that blood gathered using a one touch laser blood lancing type glucose measurement device according to the present invention is absorbed into a strip.

As shown in FIGs. 1 to 3, a one touch laser lancing type blood glucose measurement device includes a laser module 101, a focusing lens 102, a protect window 103, a controlling unit 112, a strip connector 104, a body unit 109, an interlock holder 105, a second sensing unit 106, a button unit 110, a finger interlock 107, a first sensing unit 108, and a displaying unit 111.

Here, the laser module 101 generates laser beams and irradiates the laser beams in a one direction to help a user gather blood in a one's finger. The laser beams generated in the laser module 101 are an Er:YAG laser technique and may gather the blood 113 in skin without a scar. The laser beams also have a wavelength of 2.94//m that has a strong absorbing power with respect to water and leaves on poisons in the body.

In the present invention, the Er: YAG laser technique is described as an embodiment. However, the present invention is not limited to the Er: YAG laser technique and a laser technique having the characteristics equal to the Er: YAG laser technique and leaving no harm in the body may be used.

Here, the focusing lens 102 converges the laser beams into a limited pricked skin part. In more detail, the focusing lens 102 is an element that converges the laser beams progressing in a predetermined direction to blood-gathering object skin, that is, a finger with an accurate focus. Thereby, the focusing lens 102 may prick to make a hole having a diameter of 0. 1mm to 0.5mm and a depth of 0.2mm to 4mm. However, to avoid painful sores by stimulation of nerve cells into the true skin, it is preferable that the focusing lens 102 may prick to make a hole having a depth of 0.5mm or less and diameter of 0.3mm or less.

The focusing lens 102 may also use a convex lens or an aspherical lens accurately to focus the laser beams to the pricked skin part.

Here, the protect window 103 protects the focusing lens 102 from foreign bodies in the direction, in which the laser beams from the focusing lens 102 progress. The protect window 103 is spaced apart from the focusing lens 102 by a predetermined distance in the laser progressing direction.

Here, the controlling unit 112 controls a blood sugar level measurement of blood input into a position close to a lateral side of the laser module 101 and the laser beams.

Here, the strip connector 104 connects to the controlling unit 112, but the strip connector 104 is formed in a position on which a strip hole and the protect window 103 are coincided with each other and inserts a strip 114. At this time, the used strips 114 and 400 will be described in detail later, referring to FIG. 4. The strip connector 104 obtains data of the blood 113 in the strip 114 to transfer the data to the controlling unit 112.

Here, the body unit 109 contains the laser module 101, the focusing lens 102, the protect window 103, the controlling unit 112, and the strip connector 104. At this time, the strip connector 104 is contained into the body unit 109, but it is evident that a gate for inserting the strip 114 is exposed to the outside from the body unit 109.

Here, the interlock holder 105 is disposed on the outside of the body unit 109, and positioned on the upper or lower side of the strip 114 inserted into the strip connector 104. The interlock holder 105 forms a hole. The hole disposes the strip hole 402 formed in the strip, the protect window 103, and the focusing lens 102 on a straight line, and then is pressed by a finger. Thereby, a state of the laser module 101 is changed into a ready state. At this time, the strip hole 402 is shown in FIG. 4.

When the state of the interlock holder is changed into the ready state, the laser beams may be generated using a button. However, when the state of the interlock holder is not changed into the ready state, the laser beams may not generated regardless of a state of the button, to protect a user. The Er: YAG laser technique leaves no harm in the body. However, when the laser beams generated by the Er: YAG laser technique are irradiated to the eyes, the eyes may be deadly damaged. Therefore, the present invention may prevent the accident in advance using the state of the interlock holder.

Here, the second sensing unit 106 presses the interlock holder 105 to sense the ready state of the laser module.

Here, the finger interlock 107 is formed on the outside of the body unit 109, prevents the laser beams generated in the laser module 101 from exposing to an external, and has a shape, which a finger is inserted into. At this time, since the finger interlock 107 may be inserted inward, it may be easy to take custody of the blood glucose measurement device of the present invention.

Here, the first sensing unit 108 senses whether the finger interlock 107 is opened. At this time, when the finger interlock 107 is damaged, the laser beams may be exposed to the external. Thus, when the finger interlock 107 is damaged, the first sensing unit 108 operates to stop the operations of the blood glucose measurement device, to function as a safety device.

Here, the button unit 110 is formed on a different position from the interlock holder 105 to operate the laser module 101. At this time, the button unit 110 is able to operate only when the interlock holder 105 is the ready state, for safety of a user.

Here, the displaying unit 111 outputs data measured by the operating of the laser module 101 by the button unit 110 and by the transferring the blood 113 obtained by the strip 114 to the controlling unit 112 through the strip connector 104.

As the above description, since a blood-gathering device and the blood glucose measurement device are included in one side and complexly formed, there is an advantage that the blood-gathering and the blood sugar level measurement are performed by a button operation of one time.

FIG. 4 is a diagram showing a strip used in a one touch laser lancing type blood glucose measurement device according to the present invention.

As shown in FIG. 4, a strip 400 inserted into the strip connector used in the present invention includes an electrode unit 401, a strip hole 402, a reagent unit (not shown), and a PP film 403.

Here, the electrode unit 401 is inserted into the strip connector 104 to transfer blood information to the controlling unit of the blood glucose measurement device.

Here, the strip hole 402 passes the laser beams generated from the laser module to irradiate the laser beams to transfer a finger and the blood of the finger to the reagent unit. At this time, preferably, a diameter of the strip hole 402 is 3mm, but the present invention is not limited to that.

Here, it is preferable that the regent unit (not shown) may measure any one of glucose which is a ferment, anti-insulin, and HbAIc. In addition, the present invention using the strip may measure at least one of keton and cholesterol.

Here, the PP (poly propylene) film 403 is included on the upper of the strip hole to prevent the blood glucose measurement device from polluting. At this time, it is preferable that the PP film 403 has a thickness of 0.05mm to 0.15mm, and it is more preferable that the PP film 403 has a thickness of 0.1mm.

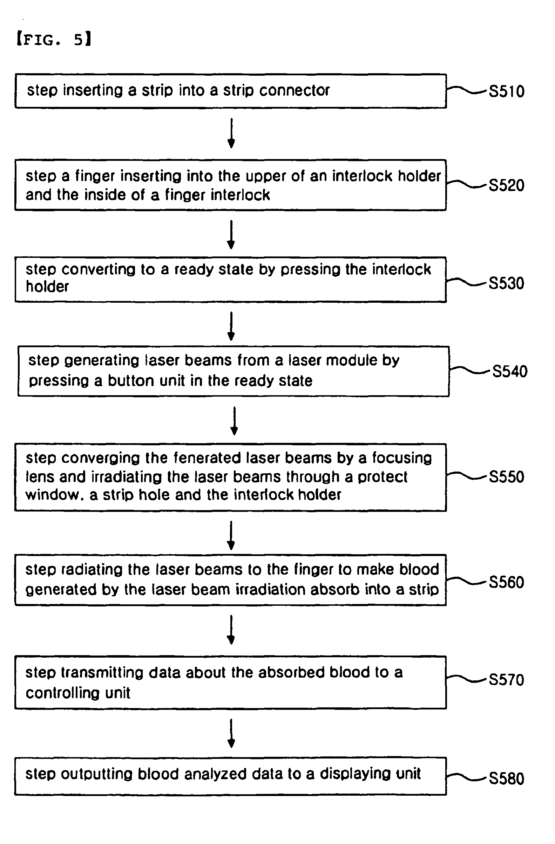

FIG. 5 is a flow chart of a blood glucose measurement method using a one touch laser lancing type blood glucose measurement according to the present invention. As shown in FIG. 5, the blood glucose measurement method using a one touch laser lancing type blood glucose measurement according to the present invention will be described below.

First, the strip is inserted into the strip connector (S510). At this time, the strip hole is coincided with the focus lens and the protect window by the strip inserted into the strip.

Sequentially, a finger is inserted into the upper of the interlock holder and the inner of the finger interlock (S520).

Sequentially, the interlock holder is pressed to be changed into a ready state (S530). At this time, in being charged into the ready state, the displaying unit displays the time when the button unit is pressed.

Sequentially, when the button unit is pressed in the ready state, laser beams are generated from the laser module (S540).

At this time, the ready state is displayed in the displaying unit, and a user presses the button unit to operate the laser module.

Sequentially, the generated laser beams is converged to the focusing lens and irradiated the laser beams through the interlock holder into the finger (S550). At this time, blood oozes from the finger by the laser beams irradiated into the finger.

In this case, the principle of the blood oozing by the laser beams is as below.

When the laser module generates the laser beams of a high temperature to irradiate into the finger, the skin of the finger is burned by the laser beams to stimulate capillaries of the inner periphery and burst them. Thereby the blood is oozed outside.

At this time, it is preferable that the temperature of the laser beams has a range of 1000<[deg.]>C to 1300<[deg.]>C. Since the laser beams had the high temperature, it is possible that the blood-gathering is performed without a disinfection operation.

Sequentially, the blood gathered by the irradiation of the laser beams is absorbed into the strip (S560).

Sequentially, data of the absorbed blood into the strip is transferred to the controlling unit. At this time, any one of glucose, anti-insulin, HbAIc may be measured (S570).

Finally, the controlling unit outputs analyzed data of the blood to the displaying unit (S580).

While this invention has been described in connection with what is presently considered to be practical exemplary embodiments, it is to be understood that the invention is not limited to the disclosed embodiments, but, on the contrary, is intended to cover various modifications and equivalent arrangements included within the spirit and scope of the appended claims.

## Claims

1. A one touch laser lancing type blood glucose measurement device comprising:
a laser module (101) generating laser beams and irradiating the laser beams in one direction;
a focusing lens (102) converging the laser beams generated from the laser module (101) and disposed in a laser irradiation direction;
a protect window (103) disposed in a laser direction progressing through the focusing lens (102) and protecting the focusing lens from foreign bodies;
a controlling unit (112) disposed on a position close to a lateral side of the laser module (101) and controlling a blood measure and the laser beams;
a strip connector (104) connected to the controlling unit (112) and formed in a position on which a strip hole (402) and the protect window (103) are coincided with each other, and inserting a strip (114);
a body unit (109) containing the laser module (101), the focusing lens (102), the protect window (103), the controlling unit (112), and the strip connector (104);
an interlock (105) disposed on the outside of the body unit (109), positioned on the upper or lower side of the strip (114) inserted into the strip connector (104), forming a hole, and changing the laser module (101) by pressing with a finger into a ready state; and
a button unit (110) formed on a lateral side different from or an opposite side to the interlock holder (105) and operating the laser module (101),
wherein, into said ready state, the interlock hole, the strip hole (402), the protect window (103) and the focusing lens (102) are disposed on a straight line.

2. The device of claim 1, further comprising a finger interlock (107) formed on the outside of the body unit (109), preventing the laser beams irradiated from the laser module (101) from exposing to an external, and having a shape, which a finger is inserted into and a first sensor (108) making a user sense the finger interlock's existence.

3. The device of claim 1, further comprising a second sensing unit (106) pressing the interlock holder to sense the ready state of the laser module (101).

4. The device of claim 1, comprising an adapted strip (114) comprising:
a reagent unit;
an electrode unit (401) inserted into a strip connector (104) to transfer blood information to the blood glucose measurement device; and
a strip hole (402) passing laser beams irradiated from said laser module (101) to irradiate the laser beams.

5. The device of claim 4, wherein said strip (114) further comprises a poly propylene film (403) included on the upper of the strip hole (402) to prevent the blood glucose measurement device from polluting.

6. The device of claim 4, the reagent unit measures any one of glucose which is a ferment, anti-insulin, and HbAIc.

7. A blood glucose measurement method comprising:
a first process (S510) inserting a strip (114) into a strip connector (104);
a second process (S520) inserting a finger into an upper of an interlock holder and into the inner of the finger lock holder;
a third process (S530) changing into a ready state by pressing the interlock holder (105);
a fourth process (S540) operating a laser module (101) using a button module (110) to generate laser beams, after the change into the ready state;
a fifth process (S550) converging the generated laser beams into a focusing lens (102), passing a protect window (103), a strip hole (402), and an interlock holder (105), and then irradiating the laser beams into the inserted finger;
a sixth process (S560) making blood oozed by the laser beams irradiated into the finger be absorbed in the strip (114);
a seventh process (S570) transmitting data for the absorbed blood to a controlling unit (112); and
an eighth process (S580) outputting blood analyzed data analyzed by the controlling unit to a displaying unit (111)
wherein, into said ready state, an interlock hole, the strip hole (402), the protect window (103) and the focusing lens (102) are disposed on a straight line.

## Patentansprüche

1. Blutzuckermessvorrichtung vom Laserlanzetten-Typ, die durch eine Berührung bedienbar ist, mit:
einem Lasermodul (101), das Laserstrahlen erzeugt und die Laserstrahlen in eine Richtung ausstrahlt;
einer Fokuslinse (102), die die Laserstrahlen, die von dem Lasermodul (101) erzeugt werden, konvergiert und in einer Laserausstrahlrichtung angeordnet ist;
einem Schutzfenster (103), das in einer Laserrichtung, die durch die Fokuslinse (102) verläuft, angeordnet ist und die Fokuslinse vor Fremdkörpern schützt;
einer Steuerungseinheit (112), die an einer Position nahe einer lateralen Seite des Lasermoduls (101) angeordnet ist, und eine Blutmessung und die Laserstrahlen steuert;
einem Streifenkonnektor (104), der mit der Steuerungseinheit (112) verbunden ist, und an einer Position geformt ist, an der ein Streifenloch (402) und das Schutzfenster (103) miteinander übereinstimmen, und einen Streifen (114) einsetzt;
einer Gehäuseeinheit (109), die das Lasermodul (101), die Fokuslinse (102), das Schutzfenster (103), die Steuerungseinheit (112) und den Streifenkonnektor (104) enthält;
einer Arretierung (105), die an der Außenseite der Gehäuseeinheit (109) angeordnet ist, die an der oberen oder unteren Seite des Streifens (114), der in den Streifenkonnektor (104) eingesetzt ist, positioniert ist, die ein Loch bildet, und das Lasermodul (101) durch Drücken mit einem Finger in einen Bereitschaftszustand versetzt; und
einer Tasteneinheit (110), die an einer lateralen Seite, die von der Arretierungshaltung (105) verschieden ist oder dieser gegenüberliegt, und das Lasermodul (101) betätigt,
wobei in dem Bereitschaftszustand die Arretierungsloch, das Streifenloch (402), das Schutzfenster (103) und die Fokuslinse (102) auf einer geraden Linie angeordnet sind.

2. Vorrichtung gemäß Anspruch 1, weiterhin mit einer Fingerarretierung (107), die an der Außenseite der Gehäuseeinheit (109) geformt ist, die verhindert, dass die Laserstrahlen, die von dem Lasermodul (101) ausgestrahlt werden, nach außen gelangen, und die eine Form, in die ein Finger eingesetzt wird und einen ersten Sensor (108) aufweist, der einem Benutzer ermöglicht, das Vorhandensein der Fingerarretierung zu erfassen.

3. Vorrichtung gemäß Anspruch 1, weiterhin mit einer zweiten Sensoreinheit (106), die die Arretierungshalterung drückt, um den Bereitschaftszustand des Lasermoduls (101) zu erfassen.

4. Vorrichtung gemäß Anspruch 1, mit einem angepassten Streifen (114), der aufweist:
eine Reagenzeinheit;
eine Elektrodeneinheit (401), die in einen Streifenkonnektor (104) eingesetzt wird, um Blutinformationen an die Blutzuckermessvorrichtung zu übertragen; und
einem Streifenloch (402), das Laserstrahlen, die von dem Lasermodul (101) ausgestrahlt werden, durchlässt, um die Laserstrahlen auszustrahlen.

5. Vorrichtung gemäß Anspruch 4, wobei der Streifen (114) weiterhin einen Polypropylenfilm (403) umfasst, der auf der Oberseite des Streifenlochs (402) umfasst ist, um die Blutzuckermessvorrichtung vor Verschmutzung zu schützen.

6. Vorrichtung gemäß Anspruch 4, wobei die Reagenzeinheit irgendeine einer Glukose, die ein Ferment, ein Anti-Insulin und ein HbAIc ist, misst.

7. Blutzuckermessverfahren, mit:
einem ersten Prozess (S510) des Einsetzens eines Streifens (114) in einen Streifenkonnektor (104);
einem zweiten Prozess (S520) des Einsetzens eines Fingers in eine Oberseite einer Arretierungshalterung und in das Innere der Fingerarretierungshalterung;
einem dritten Prozess (S530) des Versetzens in einen Bereitschaftszustand durch Drücken der Arretierungshalterung (105);
einem vierten Prozess (S540) des Betreibens eines Lasermoduls (101) unter Verwendung eines Tastenmoduls (110), um Laserstrahlen zu erzeugen, nach einem Wechsel in den Bereitschaftszustand;
einem fünften Prozess (S550) des Konvergierens der erzeugten Laserstrahlen in einer Fokuslinse (102), des Durchlaufens eines Schutzfensters (103), eines Streifenlochs (402) und einer Arretierungshalterung (105), und dann des Ausstrahlens des Laserstrahls auf den eingesetzten Finger;
einem sechsten Prozess (S560), der dazu führt, dass Blut, das dadurch austritt, dass die Laserstrahlen auf den Finger ausgestrahlt werden, in dem Streifen (114) absorbiert wird;
einem siebten Prozess (S570) des Übertragens von Daten für das absorbierte Blut an eine Steuerungseinheit (112); und
einem achten Prozess (S580) des Ausgebens von Blutanalysedaten, die durch die Steuerungseinheit analysiert werden, an eine Anzeigeeinheit (111),
wobei in dem Bereitschaftszustand ein Arretierungsloch, das Streifenloch (402), das Schutzfenster (103) und die Fokuslinse (102) auf einer geraden Linie angeordnet sind.

## Revendications

1. Dispositif de mesure du glucose sanguin de type autopiqueur laser à une touche, comprenant :
un module laser (101) générant des faisceaux laser et faisant irradier les faisceaux laser dans une direction ;
une lentille de focalisation (102) faisant converger les faisceaux laser générés à partir du module laser (101) et disposée dans une direction d'irradiation laser ;
une fenêtre de protection (103) disposée dans une direction du laser évoluant au travers de la lentille de focalisation (102) et protégeant la lentille de focalisation contre les corps étrangers ;
une unité de commande (112) disposée sur une position proche d'un côté latéral du module laser (101) et commandant une mesure sanguine et les faisceaux laser ;
un connecteur à bande (104) raccordé à l'unité de commande (112) et formé dans une position dans laquelle un trou de bande (402) et la fenêtre de protection (103) coïncident l'un avec l'autre, et insérant une bande (114) ;
une unité de corps (109) contenant le module laser (101), la lentille de focalisation (102), la fenêtre de protection (103), l'unité de commande (112) et le connecteur à bande (104) ;
un dispositif de verrouillage (105) disposé sur l'extérieur de l'unité de corps (109), placé sur le côté supérieur ou inférieur de la bande (114) insérée dans le connecteur à bande (104), formant un trou et faisant passer le module laser (101), par une pression avec un doigt, à un état prêt à l'emploi ; et
une unité de touche (110) formée sur un côté latéral différent de ou sur un côté opposé au support du dispositif de verrouillage (105) et mettant en fonctionnement le module laser (101),
dans lequel, dans ledit état prêt à l'emploi, le trou du dispositif de verrouillage, le trou de bande (402), la fenêtre de protection (103) et la lentille de focalisation (102) sont disposés en une ligne droite.

2. Dispositif selon la revendication 1, comprenant en outre un blocage de doigt (107) formé sur l'extérieur de l'unité de corps (109), empêchant les faisceaux laser qui irradient à partir du module laser (101) d'être exposés à l'extérieur et ayant une forme dans laquelle un doigt est inséré et un premier capteur (108) permettant à un utilisateur de sentir l'existence d'un blocage du doigt.

3. Dispositif selon la revendication 1, comprenant en outre une deuxième unité de capteur (106) appuyant sur le support du dispositif de verrouillage pour faire percevoir l'état prêt à l'emploi du module laser (101).

4. Dispositif selon la revendication 1, comprenant une bande adaptée (114) comprenant :
une unité de réactif ;
une unité d'électrode (401) insérée dans un connecteur à bande (104) pour transférer l'information sanguine au dispositif de mesure du glucose sanguin ; et
un trou de bande (402) faisant passer les faisceaux laser dudit module laser (101) pour faire irradier les faisceaux laser.

5. Dispositif selon la revendication 4, dans lequel ladite bande (114) comprend en outre un film de polypropylène (403) inclus sur la partie supérieure du trou de bande (402) pour préserver le dispositif de mesure du glucose sanguin d'une pollution.

6. Dispositif selon la revendication 4, dans lequel l'unité de réactif mesure un glucose qui est un ferment, une anti-insuline et HbAIc.

7. Procédé de mesure du glucose sanguin comprenant :
un premier processus (S510) d'insertion d'une bande (114) dans un connecteur à bande (104) ;
un deuxième processus (S520) d'insertion d'un doigt dans une partie supérieure d'un support du dispositif de verrouillage et dans l'intérieur du support de blocage de doigt ;
un troisième processus (S530) de passage à un état prêt à l'emploi en appuyant sur le support du dispositif de verrouillage (105) ;
un quatrième processus (S540) faisant fonctionner un module laser (101) en utilisant un module à touche (110) pour générer des faisceaux laser, après le passage à l'état prêt à l'emploi ;
un cinquième processus (S550) faisant converger les faisceaux laser générés dans une lentille de focalisation (102), passant dans une fenêtre de protection (103), un trou de bande (402) et un support de dispositif verrouillage (105), puis irradiant avec les faisceaux laser, le doigt inséré ;
un sixième processus (S560) faisant en sorte que le sang suintant du fait que les faisceaux laser irradient le doigt, soit absorbé dans la bande (114) ;
un septième processus (S570) transmettant les données relatives au sang absorbé à l'unité de commande (112) ; et
un huitième processus (S580) envoyant les données analysées du sang par l'unité de commande à une unité d'affichage (111)
dans lequel, dans ledit état prêt à l'emploi, un trou de verrouillage, le trou de la bande (402), la fenêtre de protection (103) et la lentille de focalisation (102) sont disposés en ligne droite.
